(19) Europäisches Patentamt
European Patent Office
Office européen des brevets

(11) Publication number: **0 416 746 A2**

# (12) EUROPEAN PATENT APPLICATION

(21) Application number: **90308587.6**

(22) Date of filing: **03.08.90**

(51) Int. Cl.5: **C07C 327/28**, C07C 327/30, A01N 37/00

The title of the invention has been amended (Guidelines for Examination in the EPO, A-III, 7.3).

(30) Priority: **12.08.89 GB 8918436**
**18.11.89 GB 8926136**

(43) Date of publication of application:
**13.03.91 Bulletin 91/11**

(84) Designated Contracting States:
**AT BE CH DE DK ES FR GB GR IT LI LU NL SE**

(71) Applicant: **SCHERING AGROCHEMICALS LIMITED**

**Hauxton Cambridge CB2 5HU(GB)**

(72) Inventor: **Richards, Ian Christopher**
**'Kimcot', Silver Street, Kendington**
**Haverhill, Suffolk(GB)**

(74) Representative: **Waldman, Ralph David et al**
**Schering Agrochemicals Limited Industrial Property Department Chesterford Park Research Station**
**Saffron Walden Essex CB10 1XL(GB)**

## (54) Fungicides containing an alkoxycarbonylvinyl group or an alkyloxyimino group.

(57) Compounds of formula I

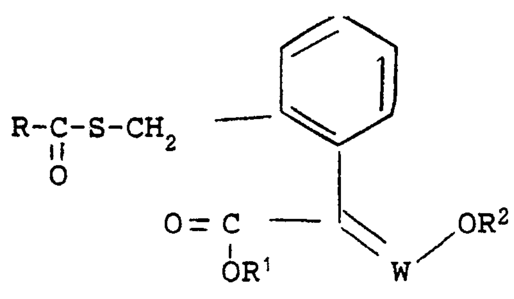

(I)

wherein W is CH or N, R is alkyl, alkenyl, alkynyl or amino, each of which is optionally substituted, aryl, heteroaryl or heterocyclyl; and $R^1$ and $R^2$ are alkyl, have fungicidal activity.

EP 0 416 746 A2

## ACRYLATE FUNGICIDES

This invention relates to compounds having fungicidal activity.

Derivatives of acrylic acid having fungicidal activity have recently been described in a number of publications, and especially EP 178826 and 203608.

According to the invention there is provided a compound of formula I

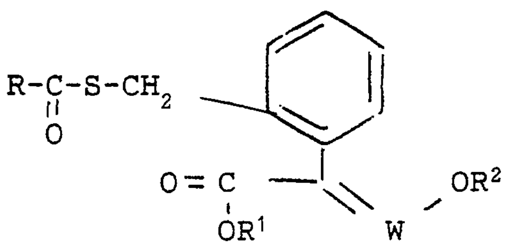

(I)

wherein

W is CH or N,

R is alkyl, alkenyl, alkynyl or amino, each of which is optionally substituted, aryl, heteroaryl or heterocyclyl; and

$R^1$ and $R^2$ are alkyl,

and acid addition salts of any compounds which are basic and basic addition salts of any compounds which are acidic.

Compounds of the invention exist as structural isomers and the invention includes individual isomers as well as mixtures of these.

$R^1$ and $R^2$ are generally $C_{1-4}$-alkyl groups, preferably methyl. When R is alkyl this is generally $C_{2-6}$-alkyl. Alkenyl and alkynyl groups are generally of three to six carbon atoms. Substituents, when present on any alkyl, alkenyl or alkynyl group, include halogen, alkoxy (e.g. of 1 to 4 carbon atoms), haloalkoxy (e.g. difluoromethoxy) hydroxy, alkylthio, nitro, optionally substituted amino, carboxy, alkoxycarbonyl, cyano, acyloxy and aryl. Aryl groups are usually phenyl, optionally substituted, e.g. by halogen, optionally substituted alkyl or alkoxy, aryl, aryloxy, nitro, amino, COOH, $COOR^2$, CN, $CONR^2R^2$ or $S(O)_nR^2$. The terms heteroaryl and heterocyclyl include groups such as thienyl, furyl, pyridyl, pyrimidinyl, pyrazolyl, thiazolyl, thiazolinyl, thiazolidinyl, oxazolyl, oxazolinyl, benzimidazolyl, tetrazolyl, benzoxazolyl, thiadiazolyl, dioxolanyl, imidazopyridinyl, 1,3-benzoxazinyl, 1,3-benzothiazinyl, oxazolopyridinyl, triazolyl, triazinyl, imidazolyl, morpholino, pyrrolidinyl, benzofuranyl, pyrazolinyl, quinolinyl, quinazolinyl, dihydroquinazolinyl or benzothiazolyl, which themselves may be substituted, e.g. as for phenyl. The term 'acyl' includes the residue of sulphonic and phosphorus containing acids as well as carboxylic acids. Acyl groups are preferably alkanoyl e.g. of 1 to 4 carbon atoms. Amino groups may be substituted, e.g. by one or two alkyl groups or two substituents can form a ring, e.g. to form a morpholino or piperidino ring. Iminomethylene groups can be substituted both on the nitrogen and carbon. Examples of substituents on the nitrogen include aryl and alkyl. Examples of substituents on the carbon include aryl, alkyl, alkylthio, alkoxy and cyano.

The compounds of the invention are particularly valuable as fungicides, especially against fungal diseases of plants, e.g. mildews and particularly cereal powdery mildew ( Erysiphe graminis ), vine downy mildew ( Plasmopara viticola ), rice blast ( Pyricularia oryzae ), cereal eyespot ( Pseudocercosporella herpotrichoides ), rice sheath blight ( Pellicularia sasakii ), grey mould ( Botrytis cinerea ), damping off ( Rhizoctonia solani ), wheat brown rust ( Puccinia recondita ), potato blight ( Phytophthora infestans ) and apple scab ( Venturia inaequalis ). Other fungi against which the compounds may be active include other powdery mildews, other rusts, and general pathogens of Deuteromycete, Ascomycete, Phycomycete or Basidiomycete origin.

The compounds of the invention also have insecticidal, acaricidal and nematicidal activity and are particularly useful in combating a variety of economically important insects, acarids and plant nematodes, including animal ectoparasites and especially Diptera, such as sheep blow-fly, Lucilia sericata , and houseflies, Musca domestica ; Lepidoptera, including Plutella xylostella , Spodoptera littoralis , Heliothis armigera and Pieris brassicae ; Homoptera, including aphids such as Megoura viciae ; Coleoptera, including corn rootworms ( Diabrotica spp., e.g. Diabrotica undecimpunctata ); and spider mites, such as Tetranychus spp..

The invention thus also provides a method of combating pests (i.e. fungi, insects, nematodes and

acarids) at a locus infested or liable to be infested therewith, which comprises applying to the locus a compound of formula I.

The invention also provides an agricultural composition comprising a compound of formula I in admixture with an agriculturally acceptable diluent or carrier.

The composition of the invention may of course include more than one compound of the invention. In addition the composition can comprise one or more additional active ingredients, for example compounds known to possess plant-growth regulant, herbicidal, fungicidal, insecticidal or acaricidal properties. Alternatively the compounds of the invention can be used in sequence with the other active ingredients.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyne-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, a dispersion, an aqueous emulsicn, a dusting powder, a seed dressing, a fumigant, a smoke, a bait, a dispersible powder, an emulsifiable concentrate or granules. Moreover it can be in a suitable form for direct application or as a concentrate or primary composition which requires dilution with a suitable quantity of water or other diluent before application.

An emulsifiable concentrate comprises a compound of the invention dissolved in a water-immiscible solvent which is formed into an emulsion with water in the presence of an emulsifying agent.

A dusting powder comprises a compound of the invention intimately mixed and ground with a solid pulverulent diluent, for example, kaolin.

A granular solid comprises a compound of the invention associated with similar diluents to those which may be employed in dusting powders, but the mixture is granulated by known methods. Alternatively it comprises the active ingredient absorbed or adsorbed on a pre-granular diluent, for example, Fuller's earth, attapulgite or limestone grit.

Wettable powders, granules or grains usually comprise the active ingredient in admixture with a suitable surfactant and an inert powder diluent such as china clay.

Another suitable concentrate is a flowable suspension concentrate which is formed by grinding the compound with water or other liquid, a wetting agent and a suspending agent.

The concentration of the active ingredient in the composition of the present invention, as applied to plants is preferably within the range of 0.001 to 3.0 per cent by weight, especially 0.01 to 1.0 per cent by weight. In a primary composition the amount of active ingredient can vary widely and can be, for example, from 5 to 95 per cent by weight of the composition.

In the method of the invention the compound is generally applied to seeds, plants or their habitat. Thus the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable application rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of

fungus as a protective measure. In both such cases the preferred mode of application is by foliar spraying. It is generally important to obtain good control of fungi in the early stages of plant growth as this is the time when the plant can be most severely damaged. The spray or dust can conveniently contain a pre- or post-emergence herbicide if this is thought necessary. Sometimes, it is practicable to treat the roots of a plant before or during planting, for example, by dipping the roots in a suitable liquid or solid composition. When the active compound is applied directly to the plant a suitable rate of application is from 0.01 to 10 kg. per hectare, preferably from 0.05 to 5 kg per hectare.

The compounds of the invention may be prepared in a variety of ways, e.g. by reacting a compound of formula II

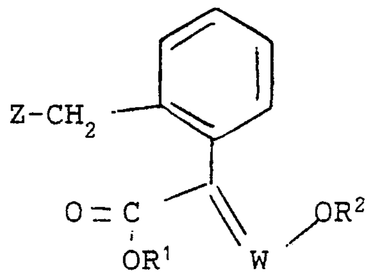

(II)

wherein Z is a leaving group, such as halogen, with a compound of formula III

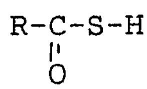

(III)

The invention is illustrated in the following Examples. Structures of isolated novel compounds were confirmed by elemental and/or other appropriate analyses. Temperatures are in °C.

Example 1

A solution of thiobenzoic acid (2.4 ml) and triethylamine (3 ml) in tetrahydrofuran (50 ml) was added to methyl (E)-3-methoxy-2-[2-(bromomethyl)phenyl]prop-2-enoate, (4.5 g). The mixture was allowed to stand at room temperature and after no more solid precipitated, the solvent was evaporated under reduced pressure. The residue was partitioned between water and ether and the ether phase was worked up to give methyl (E)-2-[2-(benzoylthiomethyl)phenyl]-3-methoxy-2-propenoate, as an oil,
(Compound 1)

Example 2

In a similar manner there was obtained:

a) from methyl (E)-3-methoxy-2-[(2-bromomethyl)phenyl]prop-2-enoate,

(i) methyl (E)-2-{2-(2,2-dimethylpropionyl)-thiomethyl]phenyl}-3-methoxy-2-propenoate, mp 79-81°, (Compound 2),

(ii) methyl (E)-2-[2-(acetylthiomethyl)phenyl]-3-methoxy-2-propenoate, mp 79-81°. (Compound 3),

(iii) methyl (E)-2-[2-(trifluoroacetylthiomethyl)-phenyl]-3-methoxy-2-propenoate. (Compound 4),

(iv) methyl (E)-2-{2-[(4-phenylbutyryl)thiomethyl]-phenyl]-3-methoxy-2-propenoate. (Compound 5),

(v) methyl (E)-2-[2-(pyrimidin-2-ylcarbonylthiomethyl)phenyl]-3-methoxy-2-propenoate. (Compound 6),

(vi) methyl (E)-2-{2-[(4-chlorobenzoyl)thiomethyl]-phenyl}-3-methoxy-2-propenoate. (Compound 7),

(vii) methyl (E)-2-{2-[(3-isopropylbenzoyl)thiomethyl]phenyl]-3-methoxy-2-propenoate. (Compound 8),

(viii)methyl (E)-2-{2-[(4-methoxybenzoyl)thiomethyl]-phenyl}-3-methoxy-2-propenoate. (Compound 9),

(ix) methyl (E)-2-{2-[(4-bromobenzoyl)thiomethyl]-phenyl}-3-methoxy-2-propenoate. (Compound 10),

(x) methyl (E)-2-[2-( o -toluoylthiomethyl)phenyl]-3-methoxy-2-propenoate. (Compound 11), and

b) from methyl [2-(bromomethyl)phenyl](3-methoxyimino)-acetate,

(iii) methyl (E)-2-[2-(benzoylthiomethyl)phenyl]-(methoxyimino)acetate, mp 79-81°. (Compound 12)

The preparation of the starting materials for these Examples is given in our EP 299694.

Test Example A

Compounds are assessed for activity against one or more of the following:

a) Foliar tests

Phytophthora infestans : late tomato blight (PI)
Plasmopara viticola : vine downy mildew (PV)
Erysiphe graminis : barley powdery mildew (EG)
Pyricularia oryzae : rice blast (PO)
Pellicularia sasakii : rice sheath blight (PS)
Botrytis cinerea : grey mould of tomato (BC)
Venturia inaequalis : apple scab (VI)

Aqueous solutions or dispersions of the compounds at the desired concentration, including a wetting agent, were applied by spray or by drenching the stem base of the test plants. These plants were then inoculated with appropriate test pathogens and kept under controlled environment conditions suitable for maintaining plant growth and development of the disease. After an appropriate time, the degree of infection of the leaf surface was visually estimated. Compounds were considered active if they gave greater than 50% control of the disease at a concentration of 125 ppm (w/v) or less.

Activities were demonstrated as follows (+ = active).

| Compound No | PI | PV | EG | PO | PS | BC | VI |
|---|---|---|---|---|---|---|---|
| 1 | + | + | | | | | + |
| 2 | + | + | + | + | + | + | |
| 3 | | | | | + | | + |
| 12 | | + | | | | | + |

## Claims

1. A compound of formula I

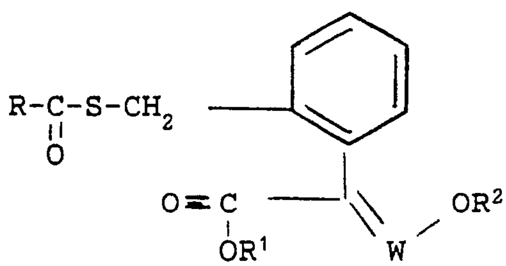

(I)

wherein
W is CH or N,

R is alkyl, alkenyl, alkynyl or amino, each of which is optionally substituted, aryl, heteroaryl or heterocyclyl; and

$R^1$ and $R^2$ are alkyl,

and acid addition salts of any compounds which are basic and basic addition salts of any compounds which are acidic.

2. An agricultural composition which comprises a compound claimed in claim 1 in admixture with an agriculturally acceptable diluent or carrier.

3. A method of combating pests at a locus infested or liable to be infested therewith, which comprises applying to the pest or its locus a compound claimed in claim 1.